# EUROPEAN PATENT APPLICATION

(11) **EP 1 988 169 A1**
(43) Date of publication of application: **05.11.2008**
(21) Application number: 08007499.0
(22) Date of filing: 17.04.2008
(51) Int. Cl.: C12P 7/06, A23K 1/06

(54) **Method for production of ethanol and fodder product**

(30) Priority: 02.05.2007 LV 070049
(71) Applicant: JP Biotechnology, SIA, Talsu rajons LV 3292 (LV)
(72) Inventor: Bosenko, Anatolij, Minsk BY-220114 (BY); Sargautis, Darius, Panevézys, LT-35107 (LT); Vaitaitis, Donats, Riga LV-1004 (LV)
(74) Representative: Dolgicere, Nina

(57) **Abstract**

The invention relates to an alcohol industry and production of protein fodder, and may find application in simultaneous production of ethanol and fodder product from cereal grain. A method for the production of ethanol and fodder product comprises steps of grain grinding, enzymatic hydrolysis and production of a wort, fermentation thereof, and steps of distillation and separation of a biomass with further drying thereof to the resultant fodder product. The object of the proposed solution is to improve the quality of the fodder product and to increase productivity of a mash rectification plant. This object is achieved by separating of the biomass from the mash before its distillation to produce a clarified mash, the separated biomass is dried together with ethanol containing therein, and the clarified mash obtained at the result of the biomass separation is subjected to distillation with the production of ethanol and a vinasse. Drying of the separated biomass is performed by means of heated inert gas with the production of a dry fodder product and water-ethanol vapor.

## Description

The invention relates to an alcohol industry and production of protein fodder, and may find application in simultaneous production of ethanol and fodder product from cereal grain.

There are known from the prior art various methods of ethanol production from grain, for example, by way of digesting an alcohol from a mash with the transferring ethanol with associated impurities into a mash distillate, rectifying ethanol to remove impurities (RU Patent 2080143, B01D 3/00). A vinasse containing proteins, cellulose, nitrogen, phosphor and micro- and macroelements, may be used for feed yeast cultivation or as fertilizers. Short shelf life of a liquid vinasse requires immediate realization thereof. This method and other similar methods do not provide alcohol-making waste processing and production of fodder for animal within the frames of embodiment of these methods.

There are also known from the prior art various methods of processing of alcohol waste in protein-rich fodder product for animals with high concentration of proteins. Method of processing waste in alcohol production according to RU Patent No. 2220195, A23K 1/06 may be cited as an example. This method involves removal of suspended colloidal and dissolved organic and mineral impurities from a vinasse by physical and chemical methods, precipitation thereof, coagulation by addition of clay yrelite, reagent of water-soluble polyelectrolyte and lime, whereafter prepared precipitate is thickened and is used as fodder product. In comparison with the prior technical solutions concerning processing of alcohol-making waste this method permits simplifying technological process of the vinasse processing into fodder products, while its embodiment constitutes a further (after production of alcohol) process , that results in product quality deterioration by interaction of a mash with proteins during the process of distillation thereof, and in additional time depending cost and separate equipment or works. Furthermore, fodder products produced by this method contain large amount of cellulose, added reagents and relatively small amount (not more than 30 %) of protein, that results in their relatively low quality.

It is more efficient to perform processing of a vinasse remaining after alcohol separation into a ready-to-use fodder product during processing of grain into ethanol.

It is known in the prior art the prototype of a method for production of an alcohol and a protein fodder from cereal grain embodied by means of apparatus according to the International application WO 2004/038031. In accordance with this method cleaned and ground grain is mixed with water, enzymatic hydrolysis is performed with the production of a wort, sugar containing in the wort is fermented and after a mash distillation, there are produced a mash distillate and a vinasse, containing suspended and dissolved dry substances (proteins, yeast cells, hemicellulose, cellulose, polypeptides, amino acids, glycerol, unfermented sugars, volatile organic acids, mineral and organic acid salts, etc.). The mash distillate is subjected to rectification resulting in production of concentrated ethanol, and the vinasse is separated by means of centrifugation into a biomass (biomass separation) and a centrifugate. The centrifugate is evaporated by bringing up the amount of dry substances up to 30 - 40 %, and after drying in the mixture with biomass separated from the vinasse, and the centrifugate is realized as a dry protein fodder (DDGS) for animals.

The disadvantages of this method are the following ones:
- deterioration in quality of protein in fodder during the mash distillation owing to its interaction with yeast metabolites, unfermented carbohydrates and other substances of the starting mash, and with new substances formed during the process of mash distillation, for example, with the products of yeast thermolysis;
- decline in productivity of a mash rectification plant owing to required often shutdowns of a mash rectification column for the purpose of cleaning, that is connected with the distillation of the mash with all insoluble and soluble dry ingredients (proteins, yeast cells, hemicelluloses, cellulose, unfermented sugars, mineral substances, etc.) and to the deposition of the part of resultant substances on the walls and trays of the mash rectification column, heat exchangers, pipelines and pumps.

It is the an object of the proposed technical solution to improve quality of protein fodder and to increase productivity of the mash rectification plant.

The set object is achieved by that in accordance with ethanol and fodder production method comprising steps of grain grinding, enzymatic hydrolysis and production of a wort, the wort fermentation, distillation steps, and the biomass separation , the last (biomass) is separated from a mash (and not from a vinasse resultant from distillation of the mash, as it is in the prototype) with the production of the clarified mash, the separated biomass is dried together with an ethanol contained therein, and the clarified mash prepared after separation of the biomass is subjected to a distillation with the production of the ethanol and the vinasse. Separated biomass containing about 20% of dry substances and 80% water-ethanol solution is dried through the use of heated inert gas, for example nitrogen or carbon dioxide (to prevent oxidation of dried components) with the production of dry fodder product and water-ethanol vapor.

Water-ethanol vapor obtained at the result of the biomass drying is condensed. Condensate is added to the clarified mash subjected to distillation, and used inert gas upon separation of water-ethanol vapor, is returned to the drying of the biomass separated from the mash.

The vinasse produced at the result of distillation of the clarified mash may be used for aerobic yeast fermentation, and yeast separated from culture liquid are dried in the mixture with the biomass separated from the mash.

The proposed method of ethanol and fodder production may be embodied through the use of both ground cereal grain and flour-and-grist mixture separated at the result of cereal grain grinding.

### Example 1 of embodiment: use of flour-and-grist mixture from cereal grain

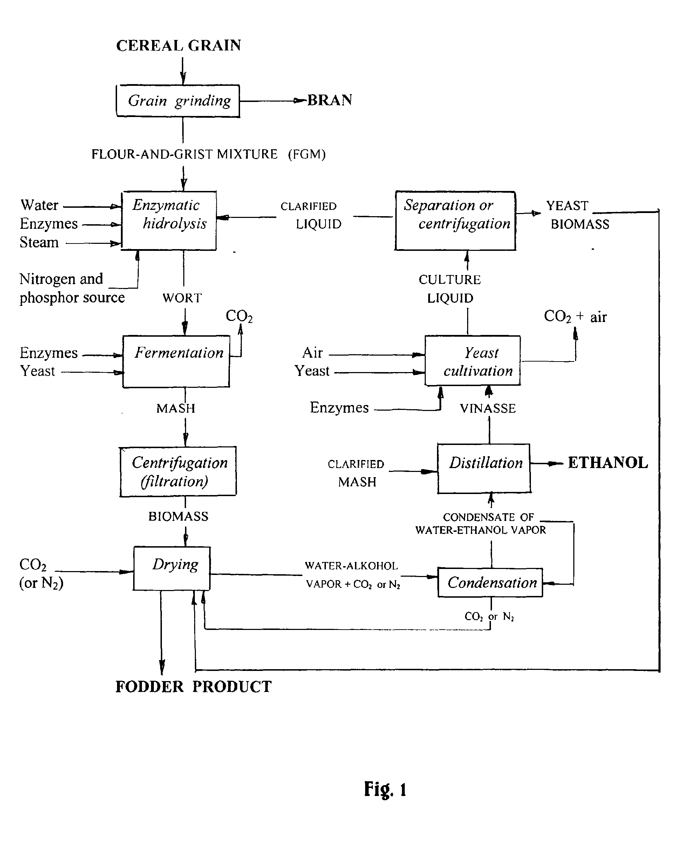

Claimed method may be illustrated by way of schematic step-to-step flowchart of ethanol and fodder production (fig. 1) comprising the step of grain grinding by means of a mill with the production of flour-and-grist mixture and bran.

In this event the embodiment of the method comprises the following steps.

### Grain grinding

Raw material (grains of wheat, triticale or rye) is freed from associated impurities and moistened to make bran elastic thereby preventing destruction thereof into small particles during grinding and inclusion into flour-and-grist mixture (hereinafter referred to as "FGM"). Moistened grains are ground by means of roller mills to prepare FGM with a particle size up to 0.8 mm and FGM yield within the range 75-80%. Resultant FGM with humidity about 15% is used to produce wort, and dry bran is used as fodder additive.

### Enzymatic hydrolysis of FGM and production of wort

FGM in the proportion of 1.0 : 2.3-2.7 is mixed with process water heated up to 66-70°C, and with enzyme preparations (xylanase, beta-gluconase and heat-stable alfa-amilase sources). Water and clarified culture liquid in different proportions are used as process water. Liquefaction of resultant suspension and FGM is performed depending on type of grain at temperature of 57-62°C during 10-15 minutes at pH 6.0 - 6.5. Liquefied suspension is continuously fed to continuous hydrolyzers , where it is heated by live steam to temperature of 85 - 95°C and FGM starch is subjected to enzymatic hydrolysis to produce dextrines. Hydrolysis time at said temperatures is 30 - 40 minutes and depends upon type of grain used. Resultant hydrolyzate is supplemented with estimated amount of mineral sources of nitrogen and phosphor, cooled to temperature up to 28 - 32°C, acidified through the use of sulfuric acid up to pH 4.6-5.0 and supplemented with estimated amount of enzymatic preparations - xylanase, beta-gluconase and heat-stable alfa-amilase sources. Resultant wort with the concentration of dissolved dry substances of 21 - 24% is used in production of mash and industrial yeast.

### Fermentation of wort and production of mash

Fermentation of a wort is performed by known methods providing deep fermentation of carbohydrates of the wort by yeast to produce ethanol, for example, by batch fermentation method.

According to this method wort cooled to temperature up to 28-32°C, with dissolved dry substances concentration of 21-24%, is fed to a batch fermenter, where seed yeast is fed concurrently in the amount of 8.0-10% of wort volume in the fermenter.

The process of the wort fermentation is performed at temperature of 28 - 35°C during 60-72 hours, while during the first 10-14 hours from the beginning of the process fermentation is performed at temperature of 29-31°C, and at the final step, starting from 45-50 hour from the beginning of the process - at temperature of 28-30°C. Wort fermentation process results in production of a mash, which contains the following dissolved and suspended substances: ethanol (12-15% volume), proteins, polypeptides, amino acids, yeast cells, hemicelluloses, glycerol, unfermented sugars, volatile organic acids, acid salts, mineral substances, small amount of cellulose, etc.) Carbon dioxide produced during wort fermentation process is withdrawn from the fermenter and is partly used as a heat-carrying medium when drying of biomass separated from mash.

### Separation of biomass from mash

Separation of the biomass from the mash is performed at temperature of 27 - 35°C by known methods, which may provide efficacious isolation of suspended dry substances and production of biomass with dry substances content not lower than 18%, for example, by means of centrifugation, at the result of which the biomass with dry substances concentration of 19 -22% and clarified mash are produced.

The biomass separated from mash contains volatile substances (water, ethanol, organic acids, etc.) and dry substances (proteins, yeast cells and small amount of amino acids , cellulose, hemicelluloses, glycerol, unfermented sugars, salts of acids, mineral substances, etc.).

### Biomass drying

Before biomass separated from mash, to which yeast may be added, is fed to drier, it is evenly mixed in a continuous mixer.

Biomass separated from mash is dried in known convection driers with the production of fodder product with moisture content not more than 10%, for example, in a fluidized bed drier. Any inert gas heated to temperature up to 180 - 200°C is used as heat-carrying medium, for example CO₂, N₂ or mixture thereof. The temperature of inert gas, for example, CO₂, and water-ethanol vapor at the exit of the drier is maintained within the range of 80 - 95°C. Fodder product and water-ethanol vapor mixture with inert gas are produced at the exit of the drier. The resultant fodder product contains 50-65% of crude protein and 1.8-2.5% of ash.

Granulated fodder product with moisture content of 8-10% is fed to packaging, while water-ethanol vapor mixture with inert gas is fed to condenser.

### Condensation of water-ethanol vapor and CO₂ regeneration

Condensation of water-ethanol vapor and CO₂ regeneration coming from the drier is performed by means of known condensers for example, a wet mixing condenser , in which condensation of water-ethanol vapor takes place due to emission of heat of this vapor to a precooled condensate of water-ethanol vapor. The water-ethanol condensate and regenerated CO₂ are produced.

Resultant condensate with ethanol concentration of 12-15% (volume) is continuously taken off from the condenser and subjected to distillation in a mixture with a clarified mash.

CO₂ separated from water-ethanol vapor is returned from the condenser to the drier, where it is reused as the heat-carrying medium in drying of the biomass.

### Distillation of the clarified mash.

Distillation of the clarified mash is performed by means of known continuous mash rectification plants to produce ethanol and vinasse.

Distillation of the clarified mash may be performed either in a mixture with the condensate of water-ethanol vapor or without addition thereof. In the first event the mixture is heated to temperature up to 70 - 80°C and after the distillation in the mash rectification plant the various purpose ethanol and the clarified vinasse are produced. In the second event more concentrated vinasse is produced. However, in this event a condensate of water-ethanol vapor is distilled in a special plant that results in complication of a technological process.

### Processing of the vinasse.

The vinasse produced at the result of distillation of the mixture of the clarified mash with the condensate of water-ethanol vapor is used for feed yeast aerobic cultivation.

Feed yeast cultivation is performed on the vinasse, to which nitrogen, phosphor sources, seeding yeast, enzymatic preparations - sources of enzymes, phytase, protease and xylanase are added. The use of phytase permits phosphoric acid and inositol to be produced from phytin that contains in the vinasse and could not be processed by yeast. The use of protease permits amino acids to be produced from peptides that contain in the vinasse and could not be processed by yeast. The use of xylanase permits monosaccharide xylose to be produced xylane that contains in vinasse and could not be processed by yeast. Resultant phosphoric acid, inositol, amino acids and xylose are additional ingredients for yeast feeding. Not only the yield of yeast from the vinasse is increased but the ingredients of the vinasse are utilized more thoroughly.

The process of yeast cultivation is performed by a continuous method at temperature of 34-36°C, pH 4.5-5.0 and aeration of enzymatic medium by sterile air. Yeast culture that extensively and thoroughly utilizes organic acids, glycerin, pentoses, hexoses and synthesizes protein with high lysine concentration is used. Cultivated yeast is produced from resultant culture liquid by means of centrifugation and is fed for drying in mixture with the biomass separated from the mash. Clarified culture liquid is fed for the step of enzymatic hydrolysis, i.e. it is used for producing the wort from FGM.

Culture liquid may be fed for the step of enzymatic hydrolysis (production of the wort from FGM) without separating yeast therefrom.

### Example 2 of embodiment: use of ground (cracked) grain

### Grinding of grain

Grinding of peeled grain (wheat, triticale, rye or corn) is performed by known methods providing the degree of fineness, that allows not less than 96% of ground grain passing through laboratory sieve with mesh diameter of 1 mm, for example, grinding by means of beater grinder.

The sequence of further operations is the same as in Example 1 of embodiment with some varieties of temperature and time mode of further steps listed below.

### Enzymatic hydrolysis and wort production.

Ground grain is mixed with heated processed water in the ratio of 1.0 : 2.5-3.0. Liquefaction of resultant suspension is performed during 15-30 minutes at temperature of 57 - 66°C: for wheat and triticale - 60-62°C, for rye - 57-59°C and for corn - 63-66°C. Duration of hydrolysis is within 60-90 minutes dependent upon kind of grain. Resultant wort with the concentration of dissolved dry substances of 20 - 23% is used in the same way as in the first event for production of the mash and industrial yeast.

### Fermentation of the wort and production of the mash

The process of the wort fermentation by yeast is performed in the same way as in Example 1 of embodiment.

### Separation of the biomass from the mash

Separation of the biomass from the mash is performed at temperature of 27 - 35°C by known methods, which may provide effective separation of suspended dry substances and production of the biomass with the content of dry substances not lower than 20%, for example, by means of centrifugation, by means of which the biomass with dry substances concentration of 20-23 % and the clarified mash are produced.

### Biomass drying

Before the biomass is fed to the drier, to which yeast cultivated on the vinasse or the evaporated vinasse or fodder product may be added, it is evenly mixed in the continuous mixer.

Biomass drying in a fluidized bed drier provides:
- the biomass drying without adding any other ingredients therein ;
- the biomass drying in the mixture with the evaporated vinasse;
- the biomass drying in the mixture with the evaporated vinasse and a part of fodder product;

### ( Evaporation of the vinasse is described below in the section «Processing of the Vinasse»)

- the biomass drying in the mixture with feed yeast cultivated on the vinasse.

In all listed variants of drying the biomass and aforementioned ingredients are evenly mixed in the continuous mixer before being fed to the drier.

Resultant fodder product contains 30-45% of crude protein and 3.5-4.2% of ash.

All steps of the the biomass drying are similar to those of Example 1 of the embodiment .

### Condensation of water-ethanol vapor and CO₂ regeneration

Upon condensation of water-ethanol vapor as it has been described in Example 1 of the embodiment the resultant condensate with ethanol concentration of 10-12% (volume) is continuously taken from the condenser and is subjected to distillation in the mixture with clarified mash.

CO₂ separated from water-ethanol vapor is returned from the condenser to the dryer, where it is reused as the heat-carrying medium in the biomass drying.

Further steps are similar to those of Example 1 of the embodiment.

### Distillation of the clarified mash

The process of distillation of clarified mash is similar to the process of distillation according to Example 1 of the embodiment.

### Processing of vinasse

The vinasse produced at the result of distillation of the mixture of the clarified mash with the condensate of water-ethanol vapor is evaporated, dried or used in cultivation of feed yeast.

Evaporation of the vinasse is performed in a continuous evaporation plant to produce the evaporated vinasse containing 30-40% of dry substances. The evaporated vinasse is dried in the mixture with the biomass separated from the mash. Secondary water condensate produced at the result of evaporation of the vinasse is used to produce the wort from ground grain.

Cultivation of feed yeast, their separation and use are performed in a similar way described in the abovementioned Example 1 of the embodiment.

Fodder product produced by claimed method has higher qualitative characteristics due to higher quality of protein therein. This is explained by the absence of negative reactions of the biomass proteins with unfermented sugars, products of metabolism and, thermolysis of yeast, which take place in known event of the distillation of the clarified mash.

According to the claimed method the productivity of the mash rectification plant is increased approximately by 7-10% due to the substantial reduction of shutdowns of the mash rectification column for cleaning, that take place during distillation of mash with all containing insoluble and soluble and dry ingredients (proteins, yeast cells, hemicellulose, cellulose, unfermented sugars, mineral substances, etc.) and deposition of a part of aforementioned and newly formed substances on the walls and trays of mash rectification column, heat-exchangers, pipelines and pumps.

## Claims

1. Method for production of ethanol and fodder product, comprising steps of grain grinding, enzymatic hydrolysis and production of a wort, its fermentation, a biomass separation and drying, and a distillation step, **characterized in that** the biomass is separated from a mash, the resultant clarified mash is subjected to distillation with the production of an ethanol and a vinasse, and the separated biomass is dried by an inert gas with production of a dry fodder product and a water-ethanol vapor.

2. Method according to claim. 1, **characterized in that** the water-ethanol vapor is condensed, and a condensate of the water-ethanol vapor is added to the clarified mash subjected to distillation.

3. Method according to claim 2, **characterized in that** the inert gas after condensation of the water-ethanol vapor is returned for reuse in the drying of the biomass separated from the mash.

4. Method according to claim 1, **characterized in that** the vinasse produced at the result of distillation of the clarified mash is used for an aerobic cultivation of yeast, for the purpose of which nutrient salts, for example, such as nitrogen and phosphor sources, cultivated yeast and enzymatic preparations are added to the vinasse, and yeast is separated from the resultant culture liquid and added to the biomass separated from the mash, and the clarified culture liquid is fed to a step of enzymatic hydrolysis.

5. Method according to claim 1, **characterized in that** the vinasse produced at the result of distillation of the clarified mash is evaporated up to the concentration of dry substances above 30%, and dried in a mixture with the biomass separated from the mash.

6. Method according to claim 5, **characterized in that** the evaporated vinasse and the biomass are dried with the addition of a part of the fodder product.
